Europäisches Patentamt

European Patent Office

Office européen des brevets

(11) Veröffentlichungsnummer: **0 472 249 A2**

# EUROPÄISCHE PATENTANMELDUNG

(21) Anmeldenummer: **91250199.6**

(22) Anmeldetag: **18.07.91**

(51) Int. Cl.5: **C02F 3/10, C12N 11/04, B01D 53/00, C02F 3/22, C02F 3/34**

(30) Priorität: **24.08.90 DE 4027220**

(43) Veröffentlichungstag der Anmeldung: **26.02.92 Patentblatt 92/09**

(84) Benannte Vertragsstaaten: **DE DK FR GB IT NL**

(71) Anmelder: **Preussag Noell Wassertechnik GmbH**
**Alfred-Nobel-Strasse 20**
**W-8700 Würzburg 1(DE)**

(72) Erfinder: **Wildenauer, Franz Xaver, Dr.**
**Dorfstrasse 1b**
**W-2861 Ritterhude(DE)**
Erfinder: **Menzel, Roman, Dr.**
**Ouellenweg 10**
**W-4450 Lingen(DE)**
Erfinder: **Vetter, Helmut**
**Oslebshauser Heerstrasse 156**
**W-2800 Bremen 21(DE)**
Erfinder: **Vorlop, Klaus-Dieter, Dr.**
**Hochstrasse 7**
**W-3300 Braunschweig(DE)**
Erfinder: **Remmers, Peter**
**Lessingplatz 6**
**W-3300 Braunschweig(DE)**

(74) Vertreter: **Kaiser, Henning**
**c/o Preussag AG, Patente und Lizenzen,**
**Postfach 15 12 27 Kurfürstendamm 32**
**W-1000 Berlin 15(DE)**

(54) Verfahren und Anlage zum biologischen Abbau von Schwefelwasserstoff.

(57) Ein biologischer Abbau von Schwefelwasserstoff erfolgt in einem Reaktor der polymerfixierte Mikroorganismen enthält, die als Biokatalysatoren in Gelkugeln eingeschlossen sind. Die Biokatalysatoren befinden sich in Wasser, und $H_2S$-Gas sowie Sauerstoff werden in einem stöchiometrischen Verhältnis zugeleitet. Durch die Mikroorganismen wird $H_2S$ in Sulfat $(SO_4^{-2})$ umgewandelt. Die Gelkugeln werden aus einer Polyvinylalkohol-Lösung, die auch die Mikroorganismen enthält, durch Eintropfen in eine tiefkalte Flüssigkeit und anschließendes sehr langsames Auftauen hergestellt, sind elastisch und abriebfest und können daher auch in Reaktoren über lange Zeit zirkulieren.

EP 0 472 249 A2

Die Erfindung bezieht sich auf ein Verfahren zum biologischen Abbau von Schwefelwasserstoff durch geeignete Mikroorganismen.

Schwefelwasserstoff kommt in technischen Gasen, wie Erdgas, Kokereigas und Abgasen aus Verbrennungsvorgängen vor, und es gibt eine Vielzahl technischer Verfahren, Schwefelwasserstoff aus diesen Gasen zu entfernen.

Ferner tritt Schwefelwasserstoff in geringen Mengen in gelöster Form in Gewässern und Abwässern sowie als Gas, beispielsweise nach der bakteriellen Zersetzung von Eiweißstoffen auf, und die vorliegende Erfindung bezieht sich insbesondere auf die Behandlung derartiger Wässer und Faulgase mittels Biokatalysatoren.

Die Anwendung von Biokatalysatoren mit in Gelkugeln immobilisierten Mikroorganismen wurde schon verschiedentlich vorgeschlagen und labormäßig durchgeführt. Gelkugeln aus Polymeren biologischer Herkunft, wie beispielsweise Alginat oder Carrageenan werden bei Scherkräften, wie sie in technischen Reaktoren vorkommen, mechanisch zerrieben. Außerdem werden sie durch in Trink- und Abwasser vorkommende Mikroorganismen angegriffen und rasch zersetzt. Ca-Alginat löst sich außerdem in Gegenwart von in geringer Konzentration im Abwasser vorkommenden Phosphat durch Ausfallung von Calcium-Phosphat auf.

Als ein sich im Abwasser inert verhaltendes synthetisches Material wurden schon Polyurethane vorgeschlagen. Die Ausgangsstoffe dieses Materials sind jedoch hochgiftig, so daß sie die einzuschließenden Mikroorganismen noch wahrend der Polymerisation abtoten oder wenigstens reduzieren. Polyurethane haben darüberhinaus eine geringe Durchlässigkeit für gelöste organische Substanzen und besitzen als weiterer Nachteil, daß sich im Inneren eines Polyurethankatalysators während der Polymerisation kaum Hohlräume ausbilden, so daß die eingeschlossenen Mikroorganismen keinen Raum zum weiteren Wachstum haben.

Die Aufgabe der Erfindung besteht darin, in gasförmigen oder flüssigen Medien einsetzbare Biokatalysatoren in Form von Kugeln oder Perlen zu schaffen, in denen Mikroorganismen eingeschlossen sind, die Schwefelwasserstoff in eine andere Schwefelverbindung umwandeln können, wobei die Kugeln eine ausreichende Stabilität und Elastizität aufweisen sollen, um auch den mechanischen Beanspruchungen in Reaktoren über lange Zeit wiederstehen zu können, und ferner in der Anwendung dieser Katalysatoren in einer geeigneten Anlage.

Es wurde gefunden, daß aus einer wässrigen Lösung eines Polyvinylalkohols mit einem Polymerisierungsgrad über 1200 und einem Hydrolysegrad von mindestens 98% mit einem Verhältnis von 40 bis 150 g Polyvinylalkohol, vorzugsweise 70 bis 100 g zu 1 l Wasser eine hochviskose Lösung hergestellt werden kann, daß aus dieser Lösung durch Eintropfen in eine tiefkalte Flüssigkeit von unter -30° C, vorzugsweise in flüssigen Stickstoff kugelförmige Körper hergestellt werden können und daß aus diesen gefrorenen Körpern bei langsamem Auftauen, insbesondere im Temperaturbereich zwischen -20° C und 5° C mit einer Erwärmungsgeschwindigkeit von nicht mehr als 20° C/h, vorzugsweise bis etwa 4° C/h zwischen -5 und +5° C stabile und elastische Gelkugeln entstehen, in denen die der Lösung zugegebenen Mikroorganismen oder ähnliche als Biokatalysator geeignete Stoffe im Polymer fixiert sind. Der hochviskosen Polyvinylalkohol-Lösung kann eine weichmachende, die Elastizität der hergestellten Gelkugeln verbessernde Komponente, wie Glyzerin oder Zucker bis zu 40 Gew.-% zugefügt werden. Als weichmachende Zusätze kommen auch wasserlösliche organische Verbindungen mit Hydroxylgruppen, wie z. B. Ethylenglycol in Frage. Die Zusätze können gleichzeitig als Nährstoffquelle für lebende Mikroorganismen dienen.

Die hergestellten Gelkugeln besitzen eine glatte abriebfeste Oberfläche und sind elastisch deformierbar. Sie werden durch in Reaktionsbehältern auftretende Scherkräfte sowie durch in dem zu behandelnden Wasser enthaltenen chemischen Bestandteile nicht zerstört.

Auch bei der Behandlung von Schwefelwasserstoff enthaltenden Gasen ist es vorteilhaft, die Biokatalysatoren in einem Reaktor in Wasser zu verwenden und die Gase gegebenenfalls mit einem Zusatz von Luft oder Sauerstoff durch den Reaktor zu leiten.

Die Sauerstoffkonzentration sollte in einem stöchiometrischen Verhältnis zu dem im Gesamtgas vorhandenen Schwefelwasserstoff stehen. Ein überstöchiometrisches Verhältnis kann von Vorteil sein.

Durch die Behandlung des Schwefelwasserstoffes mit Biokatalysatoren, vorzugsweise in Gegenwart von Sauerstoff, gegebenenfalls auch von Kohlendioxid und/oder Essigsäure, wird der Schwefelwasserstoff $H_2S$ in Sulfat $SO_4^{-2}$ umgewandelt. Das Verfahren wird vorzugsweise kontinuierlich in einem Reaktor betrieben und die mit Sulfat zunehmend belastete Flüssigkeit wird aus dem Reaktor kontinuierlich abgezogen und durch Frischwasser ersetzt. Als Reaktionsbehälter wird eine Blasensäule mit Strömungsschlaufen bevorzugt.

Zweckmäßigerweise werden in das die Biokatalysatoren enthaltende Wasser Spurenelemente und/oder Nährsalze, die für die Aktivität der Mikroorganismen erforderlich sind, eindosiert.

Bei den für die Behandlung von Schwefelwasserstoff in Frage kommenden Mikroorganismen kann unter gewissen Umständen ein übermäßiges Wachstum eintreten, so daß es zweckmäßig ist, Reaktionsbedingungen einzustellen, die das Wachstum der Mikroorganismen begrenzen, ohne jedoch deren katalytischen Aktivität einzuschränken. Die Einstellung der Reaktionsbedingungen kann über den pH-Wert und die Temperatur erfolgen.

In weiterer Ausgestaltung des erfindungsgemäßen Verfahrens ist vorgesehen, daß die mit Sulfat belastete Flüssigkeit aus dem Reaktor einer Fällung unterworfen wird. Hierzu wird die Flüssigkeit in einen Fällungsreaktor eingeleitet, in welchem durch Beimischung eines Fällungsmittels eine unlösliche Sulfatverbindung aus der Flüssigkeit ausgefällt wird.

Sofern die Sulfatverbindung nicht anderweitig verwendbar ist, wird sie zweckmäßigerweise mit Faulschlamm aus einem kommunalen Faulturm oder einer ähnlichen Anlage vermischt und mit diesem gemeinsam mittels einer Presse oder einem Dekanter entwässert.

Die unlösliche Sulfatverbindung kann auch in das Belebungsbecken einer Kläranlage eingeleitet werden, wo sie die pH-Kapazität und die Absetzeigenschaften des Belebtschlammes verbessert.

Eine Anlage zur Durchführung des Verfahrens umfaßt wenigstens einen Reaktionsbehälter in Form einer an sich bekannten Blasensäule mit einer internen oder externen Strömungsschlaufe. In diesen Reaktor wird Gas oder Flüssigkeit, die Schwefelwasserstoff enthält sowie Sauerstoff und gegebenenfalls Frischwasser von unten eingeleitet, so daß sie die Biokatalysatorkugeln in dem wässrigen Medium in einer Strömung bewegen. Die das Sulfat enthaltende Flüssigkeit wird oben aus dem Reaktor abgezogen, und einem Fällungsreaktor zugeführt, wo eine Fällung des Sulfats beispielsweise mittels Kalkmilch erfolgt.

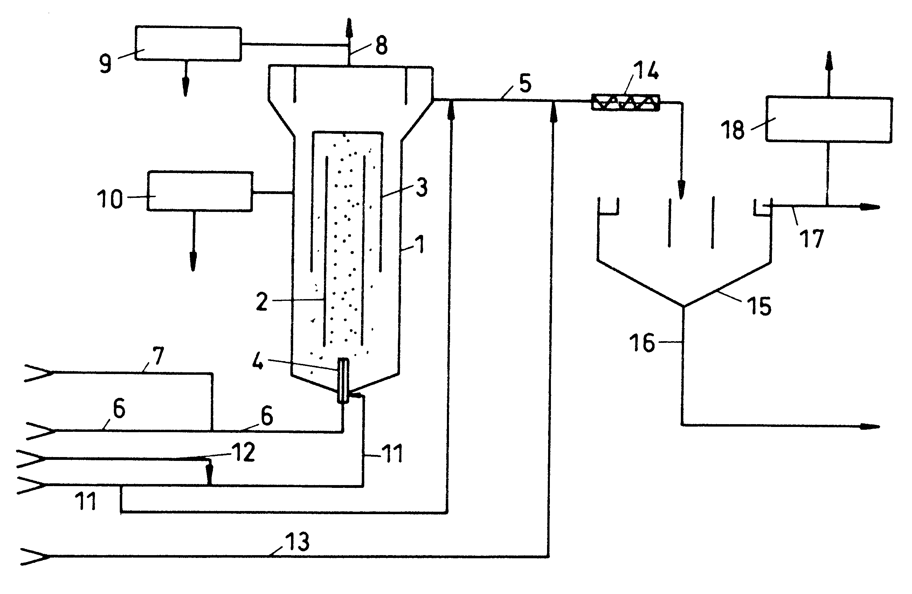

Weitere Einzelheiten werden anhand des auf der beigefügten Zeichnung schematisch dargestellten Ausführungsbeispiels beschrieben. Der gezeigte Reaktor 1 weist in seinem Inneren Einbauten auf, und zwar ein mittleres Rohr 2, das an beiden Seiten offen ist und ein weiteres Rohr 3, welches das mittlere Rohr teilweise umgibt und oben geschlossen ist. Auf diese Weise entsteht ein schlaufenförmiger Strömungsweg. Am unteren Ende des Reaktors 1 befindet sich eine Eintrittsdüse 4, durch die beispielsweise $H_2$S-haltiges Gas und Frischwasser eingeleitet wird. Das Frischwasser steigt mit dem Gas, welches sich im Wasser löst oder in kleinen Blasen dispergiert ist, in dem inneren Rohr auf, fließt zwischen den Rohren 2 und 3 wieder nach unten und steigt dann zwischen dem Rohr 3 und der Wand des Behälters 1 in das Oberteil des Reaktors auf, von wo es durch die Leitung 5 abfließt. Bei Beginn des Verfahrens befinden sich die kugelförmigen Biokatalysatoren im unteren Teil des Reaktors 1. Infolge der Strömung werden sie innerhalb des inneren Rohres 2 nach oben befördert und sinken nach der Strömungsumkehr außerhalb des Rohres 3 wieder nach unten ab. Durch diesen steten Kreislauf der Biokatalysatoren erfolgt eine gute Vermischung mit dem wässrigen Medium.

Dem über die Leitung 6 in den Reaktor 1 zuströmenden $H_2$S-haltigem Gas wird vor dem Eintritt in den Reaktor Luft oder Sauerstoff in einer etwa stöchiometrischen Menge zugemischt. Der Reaktor 1 ist mit einer Abluftleitung 8 versehen, in der eine Messung der Sauerstoffkonzentration der Abluft mittels eines Meßgerätes 9 erfolgt. Ausgehend von dieser Messung wird die Menge der über die Leitung 7 zuzuführende Luft eingestellt. An dem Behälter 1 ist weiterhin eine Meßeinrichtung 10 für eine pH-Wertmessung, in dem zuvor durch Biokatalysatoren behandelten wässrigen Medium, das Sulfat enthält. Durch die Überwachung des pH-Wertes werden die Reaktionsbedingungen entsprechend den verwendeten Mikroorganismen überwacht. Über die Messung des pH-Wertes kann die Zufuhr von Frischwasser über eine Leitung 11 in den Reaktor 1 geregelt werden. Dem Frischwasser können aus einer Leitung 12 Nährsalze für die Mikroorganismen zugefügt werden.

Dem von Schwefelwasserstoff befreiten, über die Leitung 5 aus dem Reaktor 1 abgeführten Wasser wird über eine Leitung 13 ein Fällungsmittel wie Kalkmilch zugefügt. Die Ausfällung erfolgt in einem Fällungsreaktor 14 und die ausgefällten, unlöslichen Sulfatverbindungen gelangen mit dem Wasser in einen Absetzbehälter 15. Aus diesem wird der Sulfatschlamm zu einer weiteren Behandlung über Leitung 16 abgezogen. Das behandelte Wasser fließt über Leitung 17 ab, in der eine weitere pH-Wertmessung durch ein Meßgerät 18 erfolgt. Über das Meßgerät 18 wird die Zugabe des Fällungsmittels aus der Leitung 13 gesteuert.

Bei einer Anlage, bei der $H_2$S-haltiges Wasser aus Gewässern oder als Abwasser zu behandeln ist, wird ebenfalls die vorstehend beschriebene Anordnung verwendet, jedoch wird das zu behandelnde Wasser über die Leitung 6 zugeleitet und das Frischwasser dient lediglich zur Einstellung des pH-wertes in dem Reaktor 1, wobei dem Frischwasser gegebenenfalls auch verdünnte Säuren oder Laugen zugemischt werden können.

Der Reaktor ist temperierbar. Er kann so abgewandelt werden, daß mehrere Strömungsschlaufen entstehen, und es können mehrere Reaktoren nacheinander durchströmt werden.

**Patentansprüche**

1. Verfahren zum biologischen Abbau von in Gasen enthaltenem Schwefelwasserstoff durch polymerfixierte Mikroorganismen, die als Biokatalysatoren in Gelkugeln eingeschlossen sind, die aus einer wässrigen Lösung eines Polyvinylalkohols mit einem Hydrolysegrad von mindestens 98% und einem Verhältnis von 40 bis 150 g Polyvinylalkohol zu 1 l Wasser durch Eintropfen dieser Lösung in eine wenigstens unter -30° C kalte Flüssigkeit und anschließendes Auftauen der gefrorenen Kugeln im Temperaturbereich von -20° C bis 5° C mit einer Erwärmungsrate von bis zu etwa 20° C/h hergestellt werden und die Mikroorganismenarten enthalten, welche in der Lage sind Schwefelwasserstoff in Gegenwart von Sauerstoff, Kohlendioxid und/oder Essigsäure zu Sulfat ($SO_4^{-2}$) umzuwandeln.

2. Verfahren nach Anspruch 1, dadurch gekennzeichnet, daß die Biokatalysatoren sich im Wasser befinden und Gase, die Schwefelwasserstoff enthalten, in das Wasser eingeleitet werden.

3. Verfahren nach Anspruch 1 oder 2, dadurch gekennzeichnet, daß dem zu behandelnden Schwefelwasserstoff enthaltenden Gas Sauerstoff oder Luft beigemischt wird, so daß die im gesamten Gas vorhandene Sauerstoffkonzentration in wenigstens einem stöchiometrischen Verhältnis zum im Gesamtgas vorhandenen Schwefelwasserstoff steht.

4. Verfahren nach einem der Ansprüche 1 bis 3, dadurch gekennzeichnet, daß mit Sulfat belastete Flüssigkeit aus einem Reaktor kontinuierlich ausgetauscht und durch Frischwasser ersetzt wird.

5. Verfahren nach einem der Ansprüche 1 bis 4, dadurch gekennzeichnet, daß die Behandlung in einem Reaktionsbehälter ausgeführt wird, der als Blasensäule mit Strömungsschlaufen ausgeführt ist.

6. Verfahren nach einem oder mehreren der Ansprüche 1 bis 5, dadurch gekennzeichnet, daß in das die Biokatalysatoren enthaltende Wasser für die Aktivität der Mikroorganismen erforderliche Spurenelemente und Nährsalze eindosiert werden.

7. Verfahren nach einem oder mehreren der Ansprüche 1 bis 6, dadurch gekennzeichnet, daß Reaktionsbedingungen eingestellt werden, die das Wachstum der Mikroorganismen begrenzen, ohne deren katalytische Aktivität einzuschränken.

8. Verfahren nach einem oder mehreren der Ansprüche 1 bis 7, dadurch gekennzeichnet, daß die mit Sulfat belastete, aus dem Reaktor entnommene Flüssigkeit in einen Fällungsreaktor eingeleitet wird, in dem durch Beimischung eines Fällungsmittels eine unlösliche Sulfatverbindung ausgefällt wird.

9. Verfahren nach Anspruch 8, dadurch gekennzeichnet, daß unlösliche Sulfatverbindung mit Faulschlamm aus einem kommunalen Faulturm vermischt und mit einer Presse oder einem Dekanter entwässert wird.

10. Verfahren nach Anspruch 8, dadurch gekennzeichnet, daß die unlösliche Sulfatverbindung in das Belebungsbecken einer Kläranlage eingeleitet wird, wo sie die pH-Kapazität und die Absetzeigenschaften des Belebtschlammes verbessert.

11. Anlage sur Durchführung eines Verfahrens nach einem oder mehreren der Ansprüche 1 bis 8, dadurch gekennzeichnet, daß sie einen Reaktionsbehälter in Form einer Blasensäule mit interner oder externer Strömungsschlaufe umfaßt, der mit Einrichtungen versehen ist, die ein Austreten der Biokatalysator-Kugeln verhindern, den Reaktorinhalt umwälzen und den Reaktor temperieren.

12. Anlage nach Anspruch 11, dadurch gekennzeichnet, daß Einbauten in dem Reaktor die Strömungsschlaufe so führen, daß eine Pfropfenströmung erreicht wird.

13. Anlage nach Anspruch 11 oder 12, dadurch gekennzeichnet, daß über eine Messung des pH-Wertes nahe der Austrittsseite des Frischwassers die Zufuhr von Frischwasser und die Einhaltung eines pH-Wertes geregelt werden.

14. Anlage nach einem Ansprüche 11 bis 13, dadurch gekennzeichnet, daß an der Entlüftung des Reaktors eine Einrichtung zur Messung des Sauerstoffes in der Abluft vorhanden ist und entsprechend dieser Messung eine Regelung der Zufuhr von Luft oder Sauerstoff zu dem Reaktor erfolgt.